# EUROPEAN PATENT APPLICATION

(11) **EP 2 682 749 A1**
(43) Date of publication of application: **08.01.2014**
(21) Application number: 13174821.2
(22) Date of filing: 03.07.2013
(51) Int. Cl.: G01N 33/543, G01N 33/569, C12N 7/00

(54) **Method for detecting analyte, and measuring kit**

(30) Priority: 03.07.2012 FI 20125768
(71) Applicant: Kulpakko, Janne, 20960 Turku (FI)
(72) Inventor: Kulpakko, Janne, 20960 Turku (FI)
(74) Representative: Turun Patenttitoimisto Oy

(57) **Abstract**

A method for detecting a presence of an analyte in a sample, in which method the presence of the analyte is detected by means of conductivity measurement.

## Description

### Field of the invention

The present invention relates to a method for detecting a presence of an analyte in a sample for analysis, and a measuring kit according to the preambles of the independent claims presented below. The invention also relates to bacteriophage having affinity to an analyte and affinity to a conductive compound for use in a method for detecting the presence of the analyte.

### Background of the invention

Typically biosensors and quick tests for detecting e.g. antigens are based on the use of antibodies. Immunochromatographic methods and ELISA (Enzyme linked immunosorbent assay) -tests are examples of quick tests which are based on the antibodies. These tests however require a separate analyzing apparatus so that analysis can be carried out. The antibodies for these tests have been produced by immunizing test animals. The production of the antibodies takes several months and the yield of the antibody is often questionable. In addition, an antigen given to the test animal can be toxic to the test animal or it can disintegrate as a result of the metabolism of the test animal before the production of the antibody. So, the production of the antibodies is often problematic, and the availability is not guaranteed.

Specific and high affinity quick tests can also be produced by using monoclonal antibodies, which only bind one epitope of the antigen. The production process of the monoclonal antibodies is also time consuming and doubtful.

The whole bacteriophages have typically been applied on magnetoelastic biosensors based on the surface plasmon resonance. In these sensors, bacteriophages attached to a magnetoelastic plate induce a bending of the plate when a target analyte has been bound with the bacteriophage. For example hazardous spores have specifically been detected from an air sample by using this method. However, these magnetoelastic biosensors are large and expensive devices, and so they are not suitable for the quick test applications.

### Summary of the Invention

The aim of the present invention is to remove the drawbacks of the prior art and to obtain an entirely novel solution for detecting a presence of an analyte in a sample for analysis.

The aim of the invention is to provide a simple and inexpensive method for detecting an analyte such as, but not limited to, a microbe, antigen or biocide. In addition, the aim of the invention is to provide a method which can be utilized in small-sized quick tests.

The aim of the invention is also to provide a method for detecting antigens without using antibodies.

In order to achieve among others the objects presented above, the invention is characterized by what is presented in the characterizing parts of the enclosed independent claims.

Some preferred embodiments of the invention will be described in the other claims.

The embodiments and advantages mentioned in this text relate, where applicable, both to the method, the measuring kit as well as to the uses according to the invention, even though it is not always specifically mentioned.

The invention relates to use of bacteriophage having affinity both to an analyte and to a conductive compound for detecting the analyte by the method, in which method a presence of the analyte is detected by means of conductivity measurement.

In a typical method according to the invention, the presence of the analyte in a sample solution is detected by means of the conductivity measurement after the sample has been arranged in contact with
- bacteriophages having affinity to the analyte, and affinity to a conductive compound,
- a surface, in which has been attached the analyte or bacteriophages having affinity to the analyte, and
- a conductive compound, which is able to bind to the surface of the bacteriophages.

In a typical method according to the invention for detecting a presence of an analyte in a sample solution:
- selected bacteriophages having affinity to the analyte and affinity to a conductive compound is added to the sample solution,
- the sample solution is arranged in contact with a surface in which has been attached the analyte or bacteriophages having affinity to the analyte,
- a conductive compound, which is able to bind to the surface of the selected bacteriophages, is added to the sample solution, and
- the presence of the analyte in the sample solution is detected by means of
   conductivity measurement.

A typical measuring kit according to the invention for detecting an analyte comprises at least
- selected bacteriophages having affinity to the analyte and affinity to a conductive compound, which bacteriophages are added in contact with a sample for analysis, and
- a surface, in which surface the analyte or bacteriophages having affinity to the analyte have been attached.

The shortened form "phage" is also used herein referring to the bacteriophage. The method according to the invention is based on the fact that one selected bacteriophage has an ability to bind with both a target analyte and a conductive compound, i.e. the bacteriophage used in the method according to the invention has affinity to the target analyte and also affinity to a conductive compound. Therefore, a presence of the target analyte can be detected simply by combining the phage display method and the use of the conductivity measurement.

It has now been found that simple and inexpensive quick tests for detecting a presence of a desired analyte can be manufactured by using bacteriophages having a conductive compound affinity and a target analyte affinity. The formation of the affinity in the surface of the bacteriophages is a simple and quick process and so the use of the bacteriophages is an inexpensive alternative for manufacturing quick tests. The use of the bacteriophages in the quick test is also cost-effective, because the purification of the bacteriophages is a straightforward process and they withstand long storage times and high temperatures. The affinity of the phages to the conductive compound makes it possible to apply simple and inexpensive conductivity measurement for indicating the presence of the analyte, wherefore there is no need for a separate and specific analyzing apparatus.

In the method according to the invention, analyte or bacteriophages having affinity to analyte have been immobilized on the surface. The analyte has been attached to the surface when the determination of the presence of the analyte is based on a competitive reaction. In one embodiment of the invention, proteins or small molecule-sized analytes such as antigens or target compounds (e.g. malathione, saxitoxin) have been immobilized on the surface in order for them to specifically catch the bacteriophages, which have said analyte affinity and conductive compound affinity, added to the sample solution and so applied in contact with the surface. In another embodiment of the invention, bacteriophages having affinity to large-sized analytes such as microbe cells or other cells have been immobilized on the surface in order for the target microbe or cell to specifically bind with those bacteriophages to the surface. After the bonding step on the surface, a conductive compound is added to the sample solution in contact with the surface, and the bonding of the conductive compound on the surface of the phages generates a change of the conductivity which can be properly quantified.

In an embodiment of the invention, the selection of bacteriophages having desired properties is carried out by using two separate biopanning processes. Typically, the affinity for the conductive compound is formed in the first biopanning process and after that the affinity for the desired analyte is formed in the second biopanning process. Once a specific bacteriophage for a target analyte has been selected by using biopanning processes, they can be used to detect the presence of this selected analyte.

The method according to the invention comprises two main steps. In the first step of the method, the selected bacteriophages having analyte affinity and conductive compound affinity are added to a sample solution in order for bacteriophages to bind with the analyte, if the analyte is present in the sample. In the second step of the method, the presence of the analyte is detected by using the analyte or bacteriophage having affinity to the analyte attached to the surface for binding the bacteriophages, and by adding a conductivity compound to the sample and measuring conductivity of the sample. When the target to be detected is a microbe or other large-sized analyte and bacteriophages having affinity to the target have been immobilized on the surface, the order of the above-mentioned method steps is not critical, i.e. they can be carried out in any order or even substantially at the same time, since the microbe or other large sized analyte will be bound with the phages added to the sample and also phages immobilized on the surface, i.e. large-sized analytes have several binding sites in their surface, in which bacteriphages can be bound. In the case of proteins, or small-sized analytes such as antigens, the method according to the invention is based on the competitive reaction, and so the addition of the selected bacteriophages having analyte affinity and conductive compound affinity to the sample has to be carried out before the sample is arranged in contact with the surface comprising also the target analyte, in order for the presence of the analyte in the sample to be detectable, i.e. phages are only bind either with analytes present in the sample or analytes immobilized on the surface. The analyte which has been bound to the surface is the same as the analyte to be detected in the case of small-sized analytes.

The method according to an embodiment of the invention typically comprises the following steps of:
- adding bacteriophages having affinity to the target and to the conductive compound in a sample solution, and allowing the phages time to bind with the target analyte if they are present,
- arranging the sample solution in contact with the surface, and allowing phages time to bind,
- washing the surface, so that unbound bacteriophages are removed,
- adding a conductive compound to the sample solution,
- washing the surface, so that the unbound conductive compound is removed, and
- measuring conductivity of the sample solution to determine the presence of the target.

Typically, the surface is prepared so that analyte or bacteriophages having affinity to large-sized analyte are passively attached to the surface, which means that they are bound to the surface with weak interaction between phages/analyte and the surface. Typically, the surface is solid and it is made of plastic material, e.g. polyethylene. The attachment of phages to the surface can for example be carried out in a carbonate or bicarbonate buffer solution at a pH value of about 9.

Bacteriophages can alternatively be bound to the surface chemically, for example 0.3 % glutaraldehyde or biotin-streptavidin complex can be used in the binding process.

In an embodiment of the invention, the non-specific binding sites of the surface are blocked, e.g. by using 2 % BSA (Bovin Serume Albumin) in order for any substance or molecules not to bind with these sites during the detection method according to the invention.

In an embodiment of the invention, analyte or bacteriophages have been immobilized on a surface of a reaction chamber such as a test tube or a reaction well of a multi well plate or the like. The analyte or bacteriophages can be attached to the bottom of the reaction chamber, to the wall or walls of the reaction chamber, or both to the bottom and the walls of the reaction chamber. In one preferred embodiment of the invention, the analyte or bacteriophages are attached to at least one wall or the bottom of the reaction chamber. A reaction chamber refers to all possible sample chambers and other plates or structures suitable for the attachment of the analyte or phages for carrying out the analysing method of the invention.

Typically, bacteriophages having affinity to the target and to the conductive compound are added to the sample solution in a form of individual separate phages. After addition of the phages, the sample solution is arranged in contact with the surface and then the surface can be washed with a washing buffer for removing unbound phages. The washing step(s) are not necessary. Typically, deionized water or low TDS (total dissolved solids) water is used a washing buffer. In case of organic samples, the washing buffer can be for example acetonitrile. In a typical embodiment of the invention, the surface is washed at least twice with the washing buffer.

In between the washing steps, only the bacteriophages which have been specifically bound to the surface remain in the reaction chamber, while the non-specific or unbound components are washed away.

After the washing step or steps, the conductive compound is added to the reaction chamber, wherefore conductive compound will also be bound with the phages added in the first step of the method if they are still present in the sample solution. After adding the conductive compound, the surface can also be washed in order for excess conductive compound, which has not bound to the bacteriophages, to be flushed out from the reaction chamber, but the washing step(s) are not necessary. The washing can be repeated twice.

In a preferred embodiment of the invention, a weak acid is also added to the sample solution after the addition of the conductive compound, because it detaches the conductive compound from the surface of the bacteriophages in order for the dissolution of the conductive compound to be accelerated, and so the measurement of the conductivity of the sample can be carried out right after other treating phases of the sample.

In a preferred embodiment of the invention, a conductive compound is a metal compound having particle size between 2 to 200 nm, more typically particle size between 2 to 150 nm, wherefore the conductive compound particles do not detach from the surface of the bacteriophages too easily and wherefore conductivity measurement which corresponds to the presence of the target can be carried out. The conductive compound used in the analysing method is selected by the selected phages and their affinity properties. In an embodiment of the invention, the conductive compound can be calcium carbonate. In another embodiment of the invention, the conductive compound can be silver nitrate.

A weak acid detaching the conductive compound can be for example acetic acid, formic acid, trichloroacetic acid or citric acid. In the case of silver nitrate as conductive compound, the use of weak acid is not required. Metal ions detached from the surface of the phages increase the conductivity of the sample solution.

One benefit of the invention is that the marking of the phages by using simple conductive compound is economically efficient. The easy purification of the marked bacteriophages and the marking without strong chemical compounds makes the method according to the invention inexpensive and suitable for use in quick tests. So, the present invention also reduces or even eliminates the use of strong chemicals in the production of the diagnostic quick tests.

In the method of the invention, the analyte which has been bound with bacteriophage is detected by the change of the conductivity of the sample solution. Conductivity of the sample can be measured easily by using an instrument for measuring electrical conductivity. Typically, conductivity of the sample solution is measured with a conductivity meter. Conductivity of a reference sample has also been measured in order to obtain a so-called background signal, to which the measurement result of the sample is compared. The measurement of the conductivity is a straightforward method for detecting analyte and for use in the quick tests. The instruments for measuring electrical conductivity are small-sized, inexpensive and they are suitable for use in an outside laboratory.

In an embodiment of the invention, the measurement of the conductivity is performed directly from the surface. This can be carried out without adding weak acid. Then, measurement can be carried out by using an impedimetric sensor.

In the method according to the invention, the interpretation of conductivity results is dependent on the structure of the surface in the second step of the method. When the analyte is a microbe or other large-sized analyte, the bacteriophages having the ability to bind analyte have been attached to the surface, and so the analytes of the sample, which have already bound with the phages having affinity to the analyte and to the conductivity compound, will be attached to the surface, which results in that the added conductive compound can also be bound. So, high conductivity means that the sample for analysis comprises the target analyte. In the case of antigens or other small-sized analytes, high conductivity value means that the analyte is not present in the sample or the concentration of the analyte in the sample is low, and respectively low conductivity value means high analyte concentration of the sample for analysis. This is a result of the competitive binding, because the analyte of the sample is bound with bacteriophages already in the first step of the method and so bacteriophages will not bind with analytes attached to the surface in the second step of the method.

In one embodiment of the invention, the bacteriophage is selected from the group which comprises T4 phage, T2 phage, M13 phage and ΦX174 (phi X 174) phage.

The method according to the invention does not require that the sample is pretreated before the analysis. However, the sample solution is typically diluted if the sample is very viscous or turbid, in order for the addition of phages and measurement of conductivity to be carried out. Respectively, the solid sample is dissolved in water or any other suitable solvent before the completion of the detection method.

The method according to the invention for detecting the desired target analyte is suitable for any kind of samples. The sample for analysis can for example be a blood sample. Also, liquid samples from e.g. the different production processes can be analyzed by using the method of the invention. The sample can e.g. be a water sample. Therefore, the method according to the invention can be used as a diagnostic tool in medicine, as well as a quality-control check in various industries.

The method according to the invention is suitable for use in detecting large-sized analytes such as microbe cells or other cells.

The method according to the invention is also suitable for use in detecting proteins, when the detection is based on the competitive reaction.

Further. the method according to the invention is suitable for use in detecting small molecule-sized analytes such as stable antigens, or chemical compounds (e.g. biocides). Examples of the small-sized analytes are malathione and saxitoxin. Typically, the small molecule-sized analyte refers to analytes having a molar mass below about 330 g/mol in the method according to the invention.

In an embodiment of the invention, the analyte is a microbe cell or any other cell. A microbe is a microscopic organism that comprises either a single cell or cell clusters. Microbes comprise for example bacteria, fungi, algae, and protozoa. The target microbe can be for example MRSA *(methicillin resistant Staphylococcus aureus), Pseudomona aeruginosa, Clostridium botulinum, Cryptosporidium* and *Plasmodium* species, *or Listeria* species such as *Listeria monocytogenes.* As an example of the other cells can be mentioned osteoblast cells.

In an embodiment of the invention, the analyte can be an antigen, or an allergen such as Ara h1 or Ara h2. In an embodiment of the invention, the analyte is a C-reactive protein. In another embodiment of the invention, the analyte can be a toxin molecule such as, but not limited to, ricin toxin, saxitoxin, staphylococcal enterotoxin B, Diphtheria toxin or Shiga toxin.

The measuring kit according to the invention comprises at least selected bacteriphages having affinity to an analyte and affinity to a conductive compound, which bacteriophages can be added to a sample for analysis, and a surface, in which surface the analyte or bacteriophages having affinity to the analyte have been attached. Typically, the surface is a wall or a bottom of the reaction chamber, e.g. test tube, wherein the measuring kit comprises a reaction chamber in which the method of the invention can be carried out. The selected bacteriophages are typically in the form of a solution in the measuring kit.

The measuring kit according to the invention can be manufactured for different target analytes, containing the selected bacteriophages which are able to detect that target. In addition, the measuring kit can comprise a required conductive compound solution, weak acid, washing buffer, and instructions for performing the detection method.

### Description of the drawings

In the following, some embodiments of the invention will be described in detail. The appended drawings are part of the description. In the drawings,
- Fig. 1: shows schematically an embodiment according to the method of the invention,
- Fig. 2: shows schematically another embodiment according to the method of the invention, and
- Fig. 3: shows a conductivity curve of a C-reactive protein relative to concentration of C-reactive protein in a solution.

### Detailed description of the invention

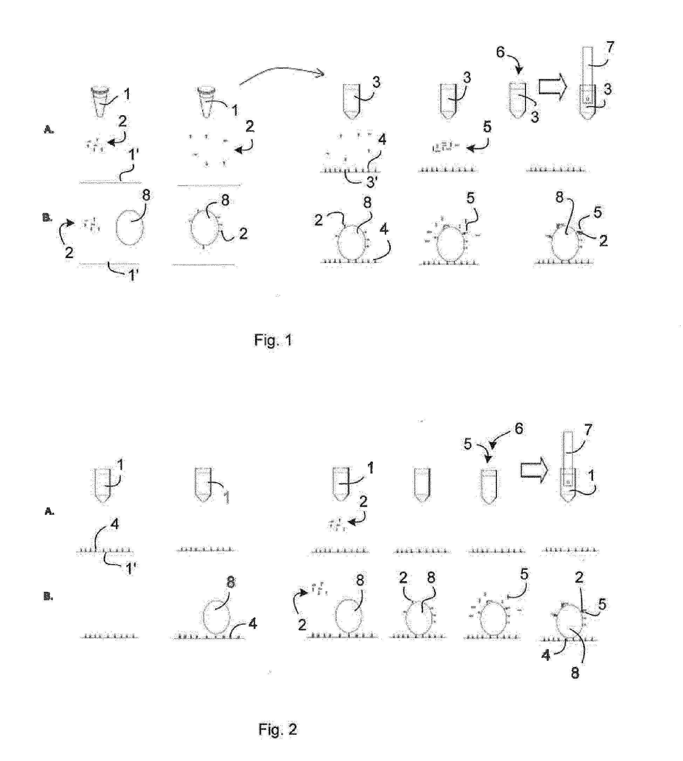

An embodiment of the invention, wherein bacteriophages are used for detecting the target microbe or any other large sized analyte having several binding sites for bacteriophages, has been schematically presented in Figure 1. Individual bacteriophages 2, which are able to bind with a target microbe and a conductive compound, are added to the first test tube 1 comprising sample solution. From the first test tube 1 the sample solution is moved to the second test tube 3. Bacteriophages 4 which bind only with target microbe have been attached to the surface 3' of the second test tube 3. A surface 1' of the first test tube does not contain any phages or analyte. The conductive compound 5 is added to the second test tube 3, and also a weak acid 6 is added to the second test tube 3 after the washing steps. Electrical conductivity of the sample solution is measured from the test tube 3 by using a conductivity meter 7.

Part A of Figure 1 illustrates an embodiment of the invention, when a sample for analysis does not contain a target microbe. Because the sample does not contain the target microbe, no microbes bind with bacteriophages 2 in the first test tube 1. Bacteriophages 2 added to the sample solution in the first test tube 1 do also not bind anywhere in the second test tube 3, but they flush out from the tube during the washing steps. Conductive compound 5 does also not bind anywhere in the test tube 3, because there are no bacteriophages having the ability to bind it, and so the conductive compound is also flushed out from the tube during the washing steps. Thus, electrical conductivity measured from the test tube 3 is low.

Part B of Figure 1 illustrates an embodiment of the invention, when a sample for analysis contains target microbes 8 (for sake of clarity, only one microbe 8 is showed in Figure). In this case, the target microbes 8 are bound with bacteriophages 2 when bacteriophages 2 are added to the first test tube 1. In the second test tube 3, the target microbes, which have already bound with phages 2 having affinity to the microbe and to the conductivity compound, are further bound with bacteriophages 4 attached to the surface 3' of the test tube 3. After the washing steps, the target microbes 8 and bacteriophages 2 bound with them in the first test tube remain in the test tube 3. Conductive compound 5 is added in an excessive amount to the test tube 3 and it is bound to the surface of the bacteriophages 2. The excess conductive compound, which has not been bound, is flushed out from the tube during the washing step. After that, a weak acid 6 is added to the test tube 3 for dissolving conductive compound 5 bound with bacteriophages 2, and so conductivity of the solution increases, which conductivity can be measured by using a conductivity meter 7.

In another embodiment of the invention (not shown in Figures), bacteriophages are used to detect antigens or any other small-sized analytes, or proteins. The presence of the target analyte in a sample is detected so that bacteriophages having affinity to the target and to the conductive compound are first added into contact with the sample in the first test tube. When the sample includes the target analyte, phages are bound with the analyte, wherein phages do not bind with analyte attached to the surface of the second test tube. The analyte attached to the surface of the second test tube is the same as the target analyte, and the selected bacteriophages are able to bind this analyte. In consequence of the washing steps, the phages bound with the analyte from the sample are flushed out from the second test tube, since they no longer bind with analytes attached to the surface. When the second test tube does not comprise bacteriophages which are able to bind to the surface of the second test tube, there are no bacteriophages which will be bound with the conductive compound added after the washing steps, and so conductivity of the sample solution is low. When the sample solution does not contain a target analyte, bacteriophages will be bound with analytes attached to the surface of the second test tube and bacteriophages will further be bound with a conductive compound added to the test tube. In this case, the sample solution has a high conductivity. A low conductivity value means that the target analyte is present in the sample, and a high conductivity value means that the sample does not comprise the target analyte.

A better understanding of the present invention may be obtained through the following examples which are set forth to illustrate, but are not to be construed as the limit of the present invention.

### Example 1: A selection of Listeria monocytogenes and calcium carbonate binding phages

### Listeria binding phages:

A fresh colony of *Listeria monocytogenes* was inoculated in 5 ml of *Listeria* Enrichment Broth according to FDA/IDF-FIL. Medium was in a 50 ml sterile conical tube. Cells were grown with vigorous shaking for 6 hours at 30°C. Next, the cell suspension was centrifugated at 5000 RPM in a GSA rotor for 8 min in sterile centrifuge bottles.

The cell pellet was washed in 250 ml of ice-cold 1x PBS as follows. First, a small amount of PBS was added to cell pellet; it was pipetted up and down or gently vortexed until cells were re-suspended. Then, the centrifuge bottle was filled with ice cold PBS and gently mixed. The cell suspension was centrifuged again at 5,000 RPM for 15 min and the supernatant was carefully poured off as soon as the rotor stopped. Cell pellet was washed in PBS avoiding that the pellet being attached to the surface of the bottle. The centrifugation time was increased if the supernatant was turbid.

The cell pellet was washed a second time by re-suspending in 250 ml of sterile ice-cold PBS using the same technique as described above. The cell suspension was centrifuged at 5000 RPM for 15 min, and the cells were recovered.

These cells were mixed gently into a phage containing solution in 1x PBS for 1 hour with shaking 100 rpm at first biopanning. 2.5 ml of this solution was filtered gently through a 0.2 µm filter. 10 ml of 1 x PBS was added through the filter to wash nonbinding phages. *Listeria* cells in membrane were removed by reverse suction with a syringe. This solution with cells and phages was added directly to a phage host strain E.coli ER2537 that was in early log-phase. The selected and amplified phages were used for panning, washing, and amplification five more times.

### Calcium carbonate binding phages:

Biopanning was conducted following a modified protocol from New England Biolabs. An aliquot (50 µl) of the ethyl methanesulfonate mutagenized wild type m13 was incubated with calcium carbonate flakes in a microcentrifuge tube. Tube contained 2 % BSA solution in 1x PBS.

To remove the unbound phage at the first biopanning process, the calcium carbonate flakes were washed 10 times with several up-down movements with 5 ml of TBST buffer [0.1% (v/v) Tween-20 in TBS (50 mM Tris-HCl, pH 7.5, 100 mM NaCl)]. After four rounds of panning, 30 washes were used with PBS and about 0.1-0.5 % (vol/vol) Tween 20. After this, flakes were added directly to bacterial host strain propagating phages.

### Co-infection: Combining Listeria and calcium carbonate binding properties:

50 ml of early log-phase E.coli ER2537 was co-infected with equal aliquots (200 µl) of both calcium carbonate and Listeria binding phages. 16 minutes after adding both phage aliquots, the cells were removed by centrifugation at 4500 g for 10 min. The supernatant was transferred to a fresh tube.

Centrifugation was repeated. 16 ml of supernatant was transferred to a new tube and 4 mL of 2.5 M NaCl/20 % PEG-8000 (w/v) was added and the mixture was briefly mixed. Phages were precipitated for 1 hr at 4 °C.

The phage was pelleted by centrifugation at 12000 g for 15 min, and then supernatant was decanted. The pellet was re-suspended in 1.5 mL of PBS, transferred to an eppendorf tube and spinned briefly to remove any cell debris.

The supernatant was transferred to a fresh tube, 200 µL of 2.5 M NaCl/20% PEG-8000 was added and the mixture was incubated on ice for 20-50 min. The supernatant was spinned at 13000 -14000 rpm in a bench top centrifuge for 10 min and then the supernatant was discarded. Remaining supernatant was removed with a pipette. The pellet was re-suspended in 250 µL of PBS.

### Screening Listeria and calcium carbonate binding phages:

To screen recombinant phages which contain both Listeria and calcium carbonate binding properties: purified bacteriophages were incubated for 15 minutes with Listeria cells 10 000 - 40 000 cells / ml in 1x PBS. After incubation 2 ml Listeria cells were washed in a syringe with 30 ml of 1x PBS. These bacteriophages were enriched in E.coli ER2537 and propagated as earlier described.

Listeria selected bacteriophages were added to a tube containing calcium carbonate flakes in PBS. The tube was incubated for 20 minutes at 20°C. Non-binding phages were removed by washing the calcium carbonate flakes 8 times with several up-down movements with 5 ml of TBST buffer [0.1% (v/v) Tween-20 in TBS (50 mM Tris-HCl, pH 7.5, 50 mM NaCl)]. These Listeria and calcium carbonate binding phages were amplified and purified as described earlier.

### Example 2: The detection of C-reactive protein (CRP) by using the method according to the invention based on the competitive reaction

The blood sample for analysis was added together with the phages having affinity to the CRP and affinity to the conductivity compound (silver nitrate) to the first test tube in 400 µl volume by a using syringe, and the sample and phages were mixed with the syringe. The sample was incubated for 4 minutes. After that, a 100 µl sample was transferred from the first test tube to the second test tube, on the surface of which has been passively attached 50 ng CRT and the non-specific binding sites of the surface had been blocked by using 2 % BSA. In addition, the second test tube included 400 µl of the PBS (Phosphate Buffered Saline) buffer solution. The sample was incubated for 3 minutes, and then the sample was washed with 600 µl of deionized water. After the washing step, silver nitrate having a particle size of 5 to 50 nm (conductive compound) was added to the sample solution in an amount of 1.5 g/ 200 µl sample, and then the sample was stayed one minute before the sample solution was again washed with the deionized water. Next, 150 µl of 15 % acetic acid (weak acid) was added to the sample solution and after two minutes the conductivity of the sample solution was measured by using a conductivity meter having a resolution of at least 0.1 µS/cm. The measuring result was compared to the zero sample, which did not contain CRP at all.

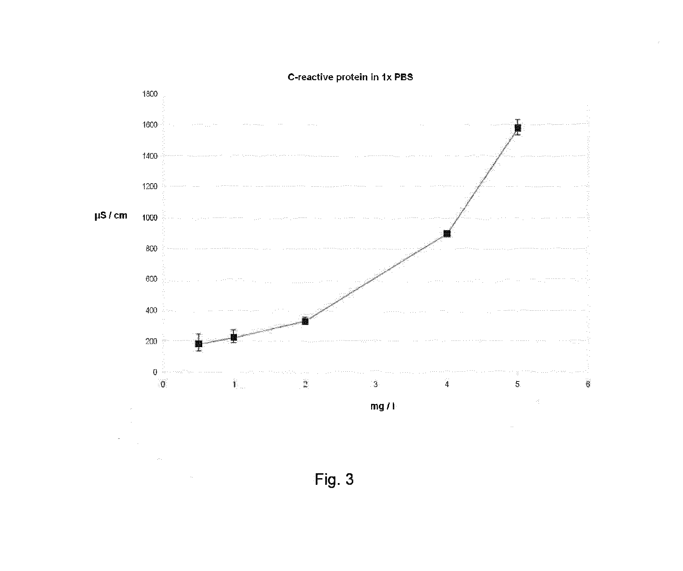

Figure 3 shows measured conductivity values (µS/cm) relative to concentration of C-reactive protein (mg/l) in the sample.

### Example 3: The detection of Listeria monocytogenes by using the method according to the invention

Figure 2 illustrates schematically a direct assay according to the invention, which is used in this example. The same reference numbers are used in Figure 1 and 2.

The sample for analysis was added to the test tube 1 in a volume of 400 µl. The 1 µg phages 4, which are able to bind with *Listeria monocytogenes* bacteria, have been passively attached to the surface 1' of the test tube 1, and the non-specific binding sites of the surface have been blocked by using 2 % BSA. The sample was incubated for 3 minutes, and then the sample was washed once with 600 µl of PBS buffer solution. Listeria cells 8 were bound with phages 4. Next, 80 ng/ 400 µl phages 2 having affinity to *Listeria monocytogenes* bacteria and affinity to silver nitrate (conductive compound) were added to the sample solution, and the sample was incubated for 4 minutes and washed with 600 µl of deionized water. After the washing step, silver nitrate 5 having a particle size of 5 to 50 nm was added to the sample solution in an amount of 1.5 g/ 200 µl sample, and then the sample was stayed one minute before the sample solution was again washed with the deionized water. Next, 150 µl of 15 % acetic acid (weak acid) 6 was added to the sample solution and after two minutes the conductivity of the sample solution was measured by using a conductivity meter 7 having a resolution of at least 0.1 µS/cm. The measuring result was compared to the zero sample, which does not contain any *Listeria monocytogenes.*

### Example 4: The detection of malathione by using the method according to the invention based on a competitive reaction

The sample for analysis comprising an organic solvent was added together with phages having affinity to malathione and affinity to the conductivity compound to the first test tube in 400 µl volume by a using syringe. The solution in the first test tube, in which the sample was added, comprises 30 % of PBS buffer solution and 70 % of acetonitrile. The solution was mixed with the syringe and it was incubated for 4 minutes. After that, a 100 µl sample was transferred from the first test tube to the second test tube, to the surface of which has been attached 1.5 µg BSA - malathione hapten. The sample was incubated for 4 minutes, and then the sample was washed with 600 µl of deionized water. After the washing step, silver nitrate (conductive compound) having a particle size of 5 to 50 nm was added to the sample solution in an amount of 1.5 g/ 200 µl sample. The sample was again washed with deionized water, and then 150 µl of 15 % acetic acid (weak acid) was added to the sample solution. After two minutes, the conductivity of the sample solution was measured by using a conductivity meter having a resolution of at least 0.1 µS/cm. The measuring result is compared to the zero sample, which did not contain malathione. When conductivity of the sample solution is low, the sample includes malathione. Respectively, if measured conductivity values are high, the sample does not comprise malathione, since phages have then been bound to the surface of the second test tube.

The invention is not restricted to the examples of the above description, but it can be modified within the scope of the inventive idea presented in the claims.

## Claims

1. A method for detecting a presence of an analyte in a sample solution, in which method
- selected bacteriophages having affinity to the analyte and affinity to a conductive compound is added to the sample solution,
- the sample solution is arranged in contact with a surface in which has been attached the analyte or bacteriophages having affinity to the analyte,
- a conductive compound, which is able to bind to the surface of the selected bacteriophages, is added to the sample solution, and
- the presence of the analyte in the sample solution is detected by means of conductivity measurement.

2. The method according to claim 1, **characterized in that** the bacteriophage is selected from the group comprising T4 phage, T2 phage, M13 phage and ΦX174 phage.

3. The method according to claim 1 or 2, **characterized in that** the bacteriophages are selected by using two separate biopanning processes so that bacteriophages having conductive compound affinity are selected in the first biopanning process, and thereafter bacteriophages, which have affinity to the analyte, are selected from these bacteriophages in the second biopanning process.

4. The method according to any of the preceding claims, **characterized in that** the conductive compound is a metal compound having a particle size between 2 to 200 nm, more typically between 2 to 150 nm.

5. The method according to any of the preceding claims, **characterized in that** after arranging the sample in contact with a surface comprising the analyte or bacteriophages having affinity to the analyte, and after adding the conductive compound, the surface is washed with a washing buffer.

6. The method according to any of the preceding claims, **characterized in that** a weak acid is added to the sample solution after the addition of the conductive compound.

7. The method according to any of the preceding claims, **characterized in that** conductivity is measured from the sample solution by using a conductivity meter, or from the surface by using an impedimetric sensor.

8. The method according to any of the preceding claims, **characterized in that** the analyte is a protein, or small molecule-sized compound, such as an antigen, and said analyte has been attached to the surface.

9. The method according to any of the preceding claims 1 to 7, **characterized in that** the analyte is a microbe cell or any other cell, and bacteriophages having affinity to said microbe cell or any other cell have been attached to the surface.

10. A bacteriophage having affinity to an analyte and affinity to a conductive compound for use in a method for detecting the analyte, in which method a presence of the analyte is detected by means of conductivity measurement.

11. The bacteriophage for use according to the claim 10, **characterized in that** the analyte is detected by the method according to any of the preceding claims 1 to 9.

12. A measuring kit for detecting an analyte, **characterized in that** it comprises at least
- selected bacteriphages having affinity to an analyte and affinity to a conductive compound, and
- a surface, in which surface the analyte or bacteriophages having affinity to the analyte have been attached.

13. The measuring kit according to claim 12, **characterized in that** the surface is at least one wall or bottom of a reaction chamber.
